(19) Europäisches Patentamt
European Patent Office
Office européen des brevets

(11) **EP 3 027 725 B1**

(12) **EUROPEAN PATENT SPECIFICATION**

(45) Date of publication and mention of the grant of the patent:
**13.07.2022 Bulletin 2022/28**

(21) Application number: **14758665.5**

(22) Date of filing: **24.07.2014**

(51) International Patent Classification (IPC):
**C12M 1/00** *(2006.01)*

(52) Cooperative Patent Classification (CPC):
**C12M 43/08; A01G 9/249; C12M 21/02; C12M 31/02;** Y02E 10/50; Y02P 20/59; Y02P 60/12

(86) International application number:
**PCT/IB2014/063368**

(87) International publication number:
**WO 2015/015372 (05.02.2015 Gazette 2015/05)**

(54) **INTEGRATED PROCESS FOR THE CULTIVATION OF ALGAE OR PLANTS AND THE PRODUCTION OF ELECTRIC ENERGY**

INTEGRIERTES VERFAHREN ZUR ZÜCHTUNG VON ALGEN ODER PFLANZEN UND ERZEUGUNG VON ELEKTRISCHER ENERGIE

PROCÉDÉ INTÉGRÉ POUR LA CULTURE D'ALGUES OU DE PLANTES ET LA PRODUCTION D'ÉNERGIE ÉLECTRIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.08.2013 IT MI20131325**

(43) Date of publication of application:
**08.06.2016 Bulletin 2016/23**

(73) Proprietor: **ENI S.p.A.**
**00144 Rome (IT)**

(72) Inventors:
• **MIGLIO, Roberta**
**I-28047 Oleggio (NO) (IT)**
• **PALMERY, Stefano**
**I-20136 Milano (IT)**

(74) Representative: **Bottero, Carlo et al**
**Barzanò & Zanardo Milano S.p.A.**
**Via Borgonuovo, 10**
**20121 Milano (IT)**

(56) References cited:
**WO-A1-2005/068605      WO-A1-2011/048458**
**US-A1- 2011 236 958      US-A1- 2012 034 679**

• **WILFRIED G J H M VAN SARK: "Luminescent Solar Concentrators A review of recent results", OPTICS EXPRESS, OSA (OPTICAL SOCIETY OF AMERICA), WASHINGTON DC, (US) , vol. 16, no. 26 22 December 2008 (2008-12-22), pages 21773-21792, XP007907393, ISSN: 1094-4087, DOI: 10.1364/OE.16.021773 Retrieved from the Internet: URL:http://www.opticsinfobase.org/abstract .cf m?URI=oe-16-26-21773**

## Description

The present invention relates to an integrated process for the cultivation of algae or plants and the production of electric energy.

Herein is also described an integrated system for the cultivation of algae or plants and the production of electric energy comprising:

- at least one luminescent solar concentrator (LSC) in which at least one photovoltaic cell (or solar cell) is positioned on at least one of its outer sides;

at least one cultivation area.

The present invention relates to an integrated process for the cultivation of algae and the production of electric energy as defined in claim 2.

The present invention also relates to an integrated process for the cultivation of plants and the production of electric energy as defined in claim 1.

Algae, in particular microalgae, are currently cultivated for the production of valuable compounds such as, for example, poly-unsaturated fatty acids [for example, eicosapentaenoic acid (EPA), docosahexaenoic acid (DHA), and the like], vitamins (for example, $\beta$-carotene, and the like) and gelling agents which fall within the nutritional, pharmaceutical and cosmetic fields.

The cultivation of microalgae for the above sectors is characterized by the relatively limited production capacities (in the order of hundreds-thousands of tons per year) and by the high added value of the compounds obtained (hundreds-thousands of euro per kilogram). For this reason, complex and expensive production systems, which must satisfy strict regulations of a sanitary and nutritional nature, typical of the above-mentioned fields, can be tolerated.

The shift from the above-mentioned fields, in which microalgae are traditionally used, to the energy field, in particular to the production of biofuels, requires the development of technologies which lead to significant increases in the production capacity and to a considerable reduction in the production costs due to the limited added value of the compounds destined for the energy field (hundreds of euro per ton).

Microalgae can in fact be used for the production of lipids, which can in their turn be used for the production of biodiesel or green diesel, or directly for the production of bio-oil or " bio-crude".

The enormous amounts of earth, water and electric energy required, make the economic and environmental sustainability of the cultivation of microalgae for producing biofuels, critical. The amount of electric energy (utility) necessary in the cultivation process of microalgae, is not only a burden from an economic point of view, but also contributes to not respecting the environmental sustainability parameters. One of the key elements for environmental sustainability is, in fact, abating the amount of electric energy deriving from a fossil source.

The cultivation process of microalgae, in fact, requires electric energy, for example, for the management of open ponds (OP), photoreactors (FR), photobioreactors (FBR), in particular for stirring the suspension of algal biomass which is formed during the growth, for the distribution of liquids and gas, and for the functioning of the equipment for the collection, concentration and conversion of the microalgae into biofuel precursors, either chemically or thermo-chemically.

It should be considered, for example, that the energy necessary for stirring an open pond (OP), which ensures: a linear rate in the order of 20 cm/sec (said rate being considered optimal for keeping the suspension of algal biomass formed, homogeneous), an effective distribution of carbon dioxide ($CO_2$), an effective oxygen release ($O_2$) and surface replacement for heat exchange, is in the order of $0.21 \text{ W/m}^2$. The overall energy necessary for the whole cultivation section, in the most favourable of cases, is in the order of 0.6 kWh/kg of algal biomass produced, which, when compared with a typical productivity of 73 t/ha/year, is equivalent to an energy consumption equal to $1.2 \text{ W/m}^2$ of surface of open pond (OP) and therefore a high energy consumption considering that this occurs in the most favourable cases. When the cultivation of microalgae is carried out for producing high added value substances, with a configuration which is energetically unfavourable, it is also economically sustainable to consume 20 kWh/kg of algal biomass produced, which, in relation to the same typical productivity mentioned above, corresponds to an energy consumption equal to $40 \text{ W/m}^2$ of surface of open pond (OP) .

Like plants, microalgae exploit solar energy for photosynthesis and consequently for their growth: it is known however that only a part of the solar energy is exploited for said photosynthesis. Processes for converting radiations of solar energy not exploited in photosynthesis into radiations that can be exploited by the same, with a consequent increase in the growth of microalgae and plants, are described in the art.

Antal T. et al., for example, in "International Journal of Hydrogen Energy" (2003), Vol. 37, pages 8859-8863, describe a study which shows that the process known as "up conversion", which takes place when a compound absorbs radiations with longer wavelengths with respect to the radiations at which it emits, can transform near-infrared radiations (NIR) into radiations which are useful for photosynthesis. Said study, however, points out that there is much work to be done before the above up conversion process can be practically exploited for obtaining an increase in photosynthesis in cyanobacteria, algae or plants.

In "Desalination" (2007), Vol. 209, pages 244-250, Hamman M. et al., describe a study relating to the evaluation of fluorescent thin films of polymethylmethacrylate impregnated with a commercial fluorescent dye, i.e. MACROLEX Fluorescent Red G, capable of concentrating solar light and of emitting it in correspondence with the absorption band of chlorophyll, i.e. 650 nm - 680 nm. In said study, it is said that these fluorescent films can be used for improving the photosynthesis of both plants grown in greenhouses and also red algae.

American patent application US 2011/0281295 describes an equipment for cultivating algae in the presence of natural light comprising: an area with a culture medium and algae that must grow (e.g., a culture tank); and a substrate in front of said area suitable for receiving solar radiation in order to photo-convert said solar radiation, said substrate comprising at least one luminescent compound capable of re-emitting radiations whose spectrum is adapted for optimizing the formation of a specific chemical compound from the photosynthesis of said algae.

US 2012/0034679 relates to a method of cultivating algae or cyanobacteria in the presence of a luminous material that converts light having a first wavelength into light having a second wavelength more suitable for use in photosynthesis of the algae or cyanobacteria and to an apparatus for implementing said method.

No mention is made in the above documents, however, of the possibility of exploiting solar radiation not only for increasing the photosynthesis but also for the contemporaneous production of electric energy.

In this respect, a photovoltaic greenhouse is known, which incorporates one or more transparent silicon thin-film photovoltaic glass panes called "Polysolar", capable of enabling photosynthesis in plants and the contemporaneous production of electric energy. Further details relating to this photovoltaic greenhouse can be found at the Internet site http://www.solarpvgreenhouse.com. As indicated in said site, however, the bronze colour of said photovoltaic glass panes is capable of allowing the passage of only 20% of visible light with a negative impact on the photosynthesis of the plants which, always in said site, is said to be minimized by the fact that the radiations closest to red, i.e. those most useful for photosynthesis, pass through said photovoltaic glass pane with a higher percentage, around 40%.

The Applicant has therefore considered the problem of finding an integrated system which enables the cultivation of algae or plants and the contemporaneous production of electric energy without negatively interfering with the growth of the same.

The Applicant has now found that the cultivation of algae or plants and the contemporaneous production of electric energy can be advantageously carried out using a system which comprises at least one luminescent solar concentrator (LSC) in which at least one photovoltaic cell (or solar cell) is positioned on at least one of its outer sides, and at least one cultivation area. Said system not only allows a good growth of algae or plants, but also protects the same from excessive exposure to ultraviolet radiations (UV radiations). Furthermore, in the case of the cultivation of algae, said system allows algae to be cultivated with a low light intensity and with a high photosynthesis yield (i.e. with a yield to algal biomass equal to that obtained with a cultivation of algae with a high light intensity). In addition, the subtraction of a part of the solar energy for the production of electric energy reduces the amount of energy reaching the liquid culture medium in which the algae are growing, and there is consequently a lower increase in the temperature of said culture medium caused by the radiations produced by said solar energy: this has a positive impact on the growth of the algae, in particular green microalgae, which is hindered by temperatures higher than 38°C.

An object of the present invention therefore relates to an integrated process for the cultivation of plants and the production of electric energy as defined in claim 1.

Another object of the present invention therefore relates to an integrated process for the cultivation of algae and the production of electric energy as defined in claim 2.

Herein described is an integrated system for the cultivation of algae or plants and the production of electric energy comprising:

- at least one luminescent solar concentrator (LSC) in which at least one photovoltaic cell (or solar cell) is positioned on at least one of its outer sides;

  - at least one cultivation area.

For the aim of the present description and of the following claims, the definitions of the numerical ranges always comprise the extremes unless otherwise specified.

For the aim of the present description and of the following claims, the term "comprising" also includes the terms "which essentially consists of" or "which consists of".

According to a preferred embodiment of the present invention, said luminescent solar concentrator (LSC) can be interposed between said cultivation area and solar light.

Preferably, said luminescent solar concentrator (LSC) may be interposed between said cultivation area and solar light so as to totally or partially cover said cultivation area.

According to a further preferred embodiment of the present invention, said luminescent solar concentrator (LSC) can be an integral part of said cultivation area and solar light.

In the process of claim 2, said cultivation area is selected from open ponds (OP), photoreactors (FR), photobioreactors (FBR), or combinations thereof.

In the invention of claim 1, said cultivation area is a greenhouse.

Preferably, in the process of claim 1, said luminescent solar concentrator (LSC) may form at least partially or totally the roof or at least partially or totally the walls of said greenhouse.

Said luminescent solar concentrator comprises at least one photoluminescent compound having an absorption range within the range of solar radiations, capable of activating photosynthesis (Photosynthetically Active Radiations - PAR.s: 400 nm - 700 nm) and an emission range capable of activating the photovoltaic cell (or solar cell). Said emission range is superimposable with respect to the maximum quantum efficiency area of the photovoltaic cell (or solar cell).

It should be pointed out that the range of radiations capable of activating photosynthesis (Photosynthetically Active Radiations - PAR.s: 400 nm - 700 nm) is exploited in different ways depending on the type of alga or plant to be cultivated. In the case of cultivations of green algae, for example, the photosynthesis is activated by solar radiations ranging from 400 nm to 500 nm (blue light) and 600 nm - 700 nm (red-orange light), whereas solar radiations within the range of 500 nm - 600 nm (green light) are not equally used for photosynthesis: in this case a photoluminescent compound which is capable of absorbing solar radiations within the range of 500 nm - 600 nm (green light), will therefore be selected.

Photoluminescent compounds which are used for the aim of the present invention are, for example, acene compounds [for example, 9,10-diphenylanthracene (DPA)] described, for example, in international patent application WO 2011/048458 in the name of the Applicant; benzothiadiazole compounds [for example, 4,7-di-2-thienyl-2,1,3-benzothiadiazole (DTB)] described, for example, in italian patent application ITMI20091796 or in international patent application WO 2012/007834, both in the name of the Applicant; benzoheterodiazole compounds disubstituted with benzodithiophene groups described, for example, in italian patent application ITMI20130605 in the name of the Applicant; naphthoheterodiazole compounds disubstituted with benzodithiophene groups described, for example, in italian patent application ITMI20130606 in the name of the Applicant; naphthothiadiazole compounds disubstituted with thiophene groups described, for example, in italian patent application ITMI20111520 in the name of the Applicant; perylene compounds known with the trade-name of Lumogen® of Basf (for example, Lumogen® F Red 305).

Said luminescent solar concentrator (LSC) comprises a matrix made of transparent material which is selected, for example, from: transparent polymers such as, for example, polymethylmethacrylate (PMMA), polycarbonate (PC), polyisobutyl methacrylate, polyethyl methacrylate, polyallyl diglycol carbonate, polymethacrylimide, polycarbonate ether, styrene acrylonitrile, polystyrene, methyl-methacrylate styrene copolymers, polyether sulfone, polysulfone, cellulose triacetate, or mixtures thereof; transparent glass such as, for example, silica, quartz, alumina, titania, or mixtures thereof. Polymethylmethacrylate (PMMA) is preferred.

Said photoluminescent compound is present in said luminescent solar concentrator (LSC) in an amount ranging from 0.1 g per surface unit to 5 g per surface unit, preferably ranging from 1 g per surface unit to 3 g per surface unit, said surface unit being referred to the surface of the matrix of transparent material expressed as m².

Said luminescent solar concentrators (LSCs) can be obtained through processes known in the art.

If, for example, the transparent matrix is of the polymeric type, said at least one photoluminescent compound can be dispersed in the polymer of said transparent matrix by, for example, dispersion in the molten state, or mass additivation, with the subsequent formation of a sheet comprising said polymer and said at least one photoluminescent compound, operating, for example, according to the so-called casting technique. Alternatively, said at least one photoluminescent compound and the polymer of said transparent matrix can be dissolved in at least one suitable solvent, obtaining a solution which is deposited on a sheet of said polymer, forming a film comprising said at least one photoluminescent compound and said polymer, operating, for example, with the use of a filmograph of the Doctor Blade type: said solvent is then left to evaporate. Said solvent can be selected, for example, from: hydrocarbons such as, for example, 1,2-dichloromethane, toluene, hexane; ketones such as, for example, acetone, acetyl acetone; or mixtures thereof.

If the transparent matrix is of the vitreous type, said at least one photoluminescent compound can be dissolved in at least one suitable solvent (which can be selected from those indicated above) obtaining a solution which is deposited on a sheet of said transparent matrix of the vitreous type, forming a film comprising said at least one photoluminescent compound operating, for example, with the use of a filmograph of the Doctor Blade type: said solvent is then left to evaporate.

Alternatively, a sheet comprising said at least one photoluminescent compound and said polymer obtained as described above, by dispersion in the molten state, or by mass additivation, and subsequent casting, can be enclosed between two sheets of said transparent matrix of the vitreous type (sandwich) operating according to the known lamination technique.

Preferably, said luminescent solar concentrator (LSC) can be produced in the form of a sheet by mass additivation and subsequent casting, as described above. Said sheets are subsequently coupled with the photovoltaic cells (or solar cells).

As indicated above, the present invention relates to an integrated process for the cultivation of algae and the production of electric energy as defined in claim 2.

Said alga can be selected from microalgae (unicellular algae). Microalgae which can be advantageously used for the aim of the present invention can be selected from the following species: *Nannochloropsis, Chlorella, Oocystis, Scenedesmus, Ankistrodesmus, Phaedactylum, Amphipleura, Amphora, Chaetoceros, Cyclotella, Cymbella, Fragilaria, Navicula, Nitzschia, Achnantes,*

*Dulaniella, Oscillatoria, Porphiridium, Traustochytrium, Spirulina,* or their consortia.

The water used for the cultivation of said alga can be selected from fresh water (e.g., river water); salt water (e.g., seawater); wastewater coming from treatment plants of civil water, or treatment plants of industrial water such as, for example, oil plants or refineries.

The cultivation of said alga can be carried out under phototrophic conditions, or under mixotrophic conditions.

The cultivation of said alga is conveniently carried out in cultivation systems known in the state of the art such as, for example, open ponds (OP), photoreactors (FR), photobioreactors (FBR), or combinations thereof.

The recovery of the algal biomass from the aqueous suspension of algal biomass can be carried out through various processes such as, for example:

- gravitational separation by means of decanters and/or thickeners, typically used in water treatment plants;
- flotation;
- gravimetric separation by means of cyclones or spirals;
- centrifugation;
- filtration by means of membranes for ultra- or micro-filtration, or vacuum filtration;

- treatment by means of filter presses or belt presses.

At the end of the above treatments, a concentrated aqueous suspension of algal biomass and water, is obtained.

In order to facilitate the concentration of the algal biomass, said aqueous suspension of algal biomass can be subjected to flocculation. Said flocculation can be carried out by means of various processes, such as, for example:

- bio-flocculation (for example, by cultivating algae in culture mediums having low nitrogen concentrations);
- addition of at least one flocculating agent to said aqueous suspension of algal biomass.

The concentration of fresh water algal strains such as, for example, the strain *Scenedesmus sp.,* can be particularly facilitated by the use of cationic polyelectrolytes, preferably polyacrylamides, used in a ratio of 2 ppm - 10 ppm.

The water released by the concentration of said aqueous suspension of algal biomass can be largely recovered and re-used in the above process as water in the production of said aqueous suspension of algal biomass (i.e. as cultivation water of algae).

Said concentrated aqueous suspension of algal biomass can be advantageously used in the production of bio-oil or bio-crude. Said bio-oil or bio-crude can be obtained, for example, by subjecting the concentrated aqueous suspension of algal biomass to liquefaction treatments, or by subjecting said concentrated aqueous suspension of algal biomass, previously dried, to pyrolysis. Said bio-oil or bio-crude can be advantageously used in the production of biofuels which can be used as such, or in a mixture with other fuels, for transportation. Or, said bio-oil or bio-crude can be used as such (biocombustible), or in a mixture with fossil combustibles (combustible oil, lignite, etc.), for the generation of electric energy or heat.

Alternatively, said concentrated aqueous suspension of algal biomass can be advantageously used in the production of lipids. Said extraction can be carried out by means of processes known in the art such as, for example, by subjecting said concentrated aqueous suspension of algal biomass, optionally previously dried, to mechanical extraction; or to extraction in the presence of carbon dioxide, or in the presence of organic solvents (for example, $C_3$-$C_8$ hydrocarbons, alcohols, or mixtures thereof), operating in liquid phase, or operating under supercritical conditions (for example, in the presence of carbon dioxide, propane, or mixtures thereof, etc.). It should be pointed out that the oily phase obtained at the end of said extraction can comprise, in addition to lipids, other compounds, such as, for example, carbohydrates, proteins, generally contained in the cell membrane of algae. Said oily phase can be subjected to hydrogenation in the presence of hydrogen and of a catalyst in order to produce "green diesel". Hydrogenation processes are known in the art and are described, for example, in european patent application EP 1,728,844.

Alternatively, said concentrated aqueous suspension of algal biomass can be advantageously used for the production of energy, for example, by subjecting the concentrated aqueous suspension of algal biomass, optionally previously dried, to heat treatments such as, for example, combustion, gasification, or partial oxidation.

The electric energy recovered by means of said luminescent solar concentrator (LSC) is used in the above-mentioned process for the cultivation of algae, for example for the management of open ponds (OP), photoreactors (FR), photobioreactors (FBR), in particular for stirring the suspension of the algal biomass formed during growth, for the distribution of liquids and gas, and for the functioning of the equipment for the collection, concentration and conversion of microalgae into precursors of biofuels, either chemically or thermo-chemically.

The present invention relates to an integrated process for the cultivation of plants and the production of electric energy as defined in claim 1.

Said plants can be selected from ornamental plants, fruit plants, vegetables.

The electric energy recovered through said luminescent solar concentrator (LSC) is used in the above-mentioned process for the cultivation of plants, for example, in the management of greenhouses, in particular for the ventilation or heating of the same.

Some illustrative and non-limiting examples are provided for a better understanding of the present invention and for its embodiment.

In the following examples:

- the 4,7-di-2-thienyl-2,1,3-benzothiadiazole (DTB) was synthesized as described in Example 1 of international patent application WO 2012/007834 in the name of the Applicant cited above;
- the 9,10-diphenylanthracene (DPA) is of Sigma-Aldrich.

## EXAMPLE 1

Preparation of a "red" luminescent solar concentrator (LSC) with photovoltaic cells

88 photovoltaic cells IXYS-KXOB22-12, each of said photovoltaic cells having a surface of 1.2 cm$^2$, were positioned at the four outer sides of an Altuglas polymethylmethacrylate (PMMA) sheet (dimensions 500 x 500 x 6 mm), obtained by mass additivation of 100 ppm of Lumogen® F Red 305 of Basf, and subsequent casting.

The photovoltaic performance of said photovoltaic cells was measured under standard lighting conditions (1.5 AM, 1000 W/m$^2$) and the current-voltage characteristics were obtained by applying an external voltage to each of said cells and measuring the photocurrent generated with a digital multimeter "Keithley 2602A" (3A DC, 10A Pulse) obtaining the following result:

- maximum power (Pmax) = 14.8 W/m$^2$.

## EXAMPLE 2

Preparation of a "yellow" luminescent solar concentrator (LSC) with photovoltaic cells

88 photovoltaic cells IXYS-KXOB22-12, each of said photovoltaic cells having a surface of 1.2 cm$^2$, were positioned at the four outer sides of an Altuglas polymethylmethacrylate (PMMA) sheet (dimensions 500 x 500 x 6 mm), obtained by the mass additivation of 100 ppm of 9,10-diphenylanthracene (DPA) and 100 ppm of 4,7-di-2-thienyl-2,1,3-benzothiadiazole (DTB), and subsequent casting.

The photovoltaic performance of said photovoltaic cells was measured under standard lighting conditions (1.5 AM, 1000 W/m$^2$) and the current-voltage characteristics were obtained by applying an external voltage to each of said cells and measuring the photocurrent generated with a digital multimeter "Keithley 2602A" (3A DC, 10A Pulse) obtaining the following result:

- maximum power (Pmax) = 12.0 W/m$^2$.

## EXAMPLE 3

Cultivation of strawberry

Two equivalent strawberry seedlings of the 4-season reflowering SELVA/Thelma and Louise type were taken and positioned, one exposed directly to solar radiation and the other through the "red" luminescent solar concentrator (LSC) obtained as described in Example 1.

During the exposure period (20 days), the average solar radiation, measured at 12.00 noon, proved to be 700 W/m$^2$. On the first test day, a solar radiation of 1,000 W/m$^2$ was registered at 12.00 noon. Of this solar radiation, the fraction ranging from 400 nm - 700 nm defines the photosynthetically active fraction ("Photosynthetically Active Radiations" - P.A.R.s), which is equal to 400 W/m$^2$, equivalent to 1840 μE/m$^2$/sec.

Under these conditions, the strawberry exposed directly to sunlight receives 1840 μE/m$^2$/sec, whereas the strawberry positioned under the abovementioned "red" luminescent solar concentrator (LSC), receives 681 μE/m$^2$/sec.

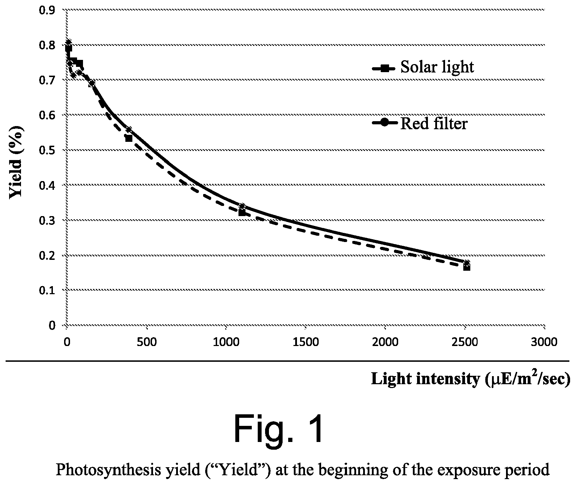

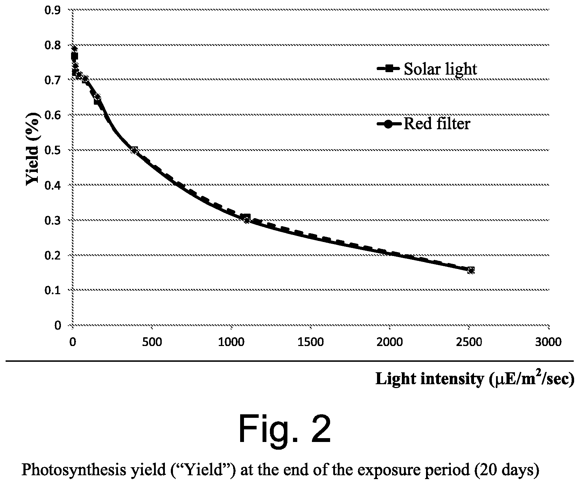

The photosynthesis parameters of the two seedlings were also measured at the beginning of the exposure period and after 20 days. The results obtained are reported in Figures 1 and 2 in which, the photosynthesis yields ["Yield"- (%)] are reported in the ordinate, and the violet light intensities emitted at 440 nm, in μE/m$^2$/sec ["Light intensity" - (μE/m$^2$/sec)], are reported in the abscissa. A MULTICOLOR-PAM "Multiple Excitation Wavelength Chlorophyll Fluorescence Analyzer" of Walz was used for these measurements.

As can be deduced from the above Figures 1 and 2, the trends of the photosynthesis yield ("Yield") for the two seedlings are superimposed both at the beginning and the end of the test, showing the same good vegetative state, with and without the "red" luminescent solar concentrator (LSC).

## EXAMPLE 4

Preparation of the algal inoculum

The algal strain of the internal collection *Nannochloropsis salina* was used, which normally grows in seawater. The cultivation process adopted is described hereunder.

A 50 ml sample of culture of *Nannochloropsis salina,* having a concentration of dry algal biomass of 0.8 g/l, previously maintained at -85°C in a solution at 10% of glycerine, was defrosted, leaving it at room temperature, and was then subjected to centrifugation to remove the supernatant, obtaining a cell paste.

The cell paste thus obtained was inoculated into a glass photobioreactor (FBR) having the following dimensions: 11 cm (length of base), 5.5 cm (width of base) and 18.5 cm (height), with a useful volume equal to 750 ml, open at the surface (not sterile), containing 350 ml of seawater to which nutrients had been added (culture medium indicated hereunder), obtaining an algal culture.

The culture medium used was the following: seawater (350 ml) having a conductivity equal to 50 mS/cm - 55 mS/cm, to which only the nitrate, phosphate and iron (III) nutrients had been added in the following amounts:

$NaNO_3$: 0.5 g/l;
$KH_2PO_4$: 0.045 g/l;
$FeCl_3$: 0.006 g/l.

The above photobioreactor was illuminated from the outside with a fluorescent lamp characterized by a solar spectrum, (of the type OSRAM Dulux D/E, 26W/840, "Lumilux cool white", temperature (T) = 4000 K, G24q-3), positioned, with respect to said photobioreactor, at such a distance so as to produce a light intensity measured on the outer surface equal to 250 $\mu E/m^2$/sec, in continuous, 24 hours a day. The light was supplied on only one side of the photobioreactor and the photosynthetically active radiations ["Photosynthetically Active Radiations" - (P.A.R.s): 400 nm - 700 nm] were measured with a QSL-2201 radiometer ("Quantum Scalar Radiometer" - QSL) of Biospherical Instruments Inc., equipped with a scalar irradiance sensor.

Said algal culture was grown at a constant temperature, equal to 23°C, and the desired temperature was obtained with a thermostatic bath and an immersed coil, in the presence of carbon dioxide ($CO_2$) diluted in nitrogen ($N_2$), which was fed to said reactor by bubbling, with a flow which was such as to maintain the pH within the range of 6.5 - 7.5.

After about a week, the algal culture had reached a concentration of dry algal biomass of 0.5 g/l. Said inoculum was used for the subsequent cultivation tests.

EXAMPLE 5

Algal cultivations with and without luminescent solar concentrators (LSCs)

The algal cultivations were carried out in pairs in 750 ml photobioreactors (FBRs), the same as those used for the cultivation of the inoculum in Example 4, assessing the growth in light after the application of the "red" luminescent solar concentrator (LSC) obtained as described in Example 1 or of the "yellow" luminescent solar concentrator (LSC) obtained as described in Example 2, with respect to a reference put under the same growth conditions but without a luminescent solar concentrator (LSC). The algal cultivations were carried out batchwise, starting from the same culture medium used for the preparation of the inoculum as described in Example 4, and inoculating the photobioreactors (FBRs) so as to initially have 50 ppm of algal biomass.

The growth measurements were integrated by measurements of the photosynthesis capacity to allow a better characterization of the effect of light on the vegetative state of the microalgae.

The following luminescent solar concentrators (LSCs) were used for the purpose:

- "yellow" luminescent solar concentrator (LSC) which absorbs blue light ($\lambda$ < 500 nm) within the range of photosynthetically active radiations;
- "red" luminescent solar concentrator (LSC) which absorbs green light (500 nm < $\lambda$ < 600 nm) within the range of photosynthetically active radiations. The following pairs of algal cultivations were carried out:

  - K141 [without a "red" luminescent solar concentrator (LSC)] and K140 [with a "red" luminescent solar concentrator (LSC)]: with the same light intensity of 250 $\mu E/m^2$/s measured on the surface of the photobioreactor (FBR) (value typical of light limiting growth) and a temperature equal to 23°C; in the case of "red" LSC, the light intensity of 250 $\mu E/m^2$/s, measured on the surface of the photobioreactor (FBR) was obtained by illuminating said "red" LSC with a light intensity of 712 $\mu E/m^2$/s;
  - K143 [without a "red" luminescent solar concentrator (LSC)] and K142 [with a "red" luminescent solar concentrator (LSC)]: with the same light intensity emitted from the source, corresponding to 865 $\mu E/m^2$/s, measured on the surface of the photobioreactor (FBR) without a LSC and corresponding to 409 $\mu E/m^2$/s measured on the surface of the photobioreactor (FBR) after passing through said "red" LSC (value typical of photoinhibition) and a temperature equal to 23°C;
  - K145 [without a "red" luminescent solar concentrator (LSC)] and K144 [with a "red" luminescent solar concentrator (LSC)]: with the same light intensity emitted from the source, corresponding to 616 $\mu E/m^2$/s, measured on the surface of the photobioreactor (FBR) without a LSC and corresponding to 317 $\mu E/m^2$/s measured on the surface of the photobioreactor (FBR) after passing through said "red" LSC (value typical of light limiting growth) and a temperature equal to 31°C;
  - K131 [without a "yellow" luminescent solar concentrator (LSC)] and K130 [with a "yellow" luminescent solar concentrator (LSC)]: with the same light intensity of 250 $\mu E/m^2$/s measured on the surface of the photobioreactor (FBR), (value typical of light limiting growth) and a temperature equal to 23°C.

The exponential growth phases, having a duration varying from 60 hours to 100 hours, were monitored for each pair of tests, carrying out one/two daily withdrawals of algal culture from each photobioreactor (FBR).

Each withdrawal was subjected to measurement of the optical density, at a wavelength equal to 610 nm, using a Hanna multiparameter photometer series 83099, in order to be able to follow the growth trend of the algal biomass.

The measurement of the optical density was correlated with the measurement of the concentration of algal biomass, calibrating the signal obtained with said optical density measurement with the measurement of the dry weight of algal biomass: the concentration of algal biomass was consequently recalculated from the direct measurement of the optical density.

The specific growth ($\mu$), associated with the light and temperature of each exponential growth phase, was recalculated by interpolating the measurements of the concentration of algal biomass with time according to the following equation (I):

$$C_{(t)} = C_{(t°)} * \exp(\mu * t) \quad (I)$$

wherein:

- $C_{(t)}$ = concentration of algal biomass at time (t) of the withdrawal (expressed in hours) (g/m$^3$);
- $C_{(t°)}$ = concentration of algal biomass at time (t°) at the beginning of the cultivation (expressed in hours) (g/m$^3$);
- $\mu$ = specific growth (sec$^{-1}$)

obtaining the following results:

- K141 [without a "red" luminescent solar concentrator (LSC)]: $\mu$ = 0.020 sec$^{-1}$;
- K140 [with a "red" luminescent solar concentrator (LSC)]: $\mu$ = 0.020 sec$^{-1}$;
- K143 [without a "red" luminescent solar concentrator (LSC)]: $\mu$ = 0.017 sec$^{-1}$;
- K142 [with a "red" luminescent solar concentrator (LSC)]: $\mu$ = 0.019 sec$^{-1}$;
- K145 [without a "red" luminescent solar concentrator (LSC)]: $\mu$ = 0.022 sec$^{-1}$;
- K144 [with a "red" luminescent solar concentrator (LSC)]: $\mu$ = 0.026 sec$^{-1}$;
- K131 [without a "yellow" luminescent solar concentrator (LSC)]: $\mu$ = 0.020 sec$^{-1}$;
- K130 [with a "yellow" luminescent solar concentrator (LSC)]: no growth is observed.

From the data indicated above, it can be deduced that there are no significant differences in behaviour with the same light energy which reaches the photobioreactor (FBR) within the spectrum useful for photosynthesis (red + blue). Green light has no effect, even if it is sent onto the cultivation, it is not used.

Photosynthesis data

Fluorescence measurements were carried out with a WATER-PAM fluorometer of Heinz Walz GmbH and analysis using Phyto-Win Rapid Light Curve software of Phyto Win, plus recovery of the photosynthesis yield [Yield - (%)] by re-adaptation to the dark following the Phyto Win software protocol.

The protocol envisages the use of photosynthetically active light with an increasing intensity up to about 2500 $\mu$E/m$^2$/sec. Each step lasted 10 seconds, eight steps were programmed and at the end of each step, a saturation pulse of a few milliseconds was sent.

The sample to be analyzed was taken from the photobioreactor (FBR) and diluted with demineralized water in order to make it suitable for the measurement instrument (Water PAM) which requires a basic fluorescence of the sample within an established range.

With respect to the tests K143 [without a "red" luminescent solar concentrator (LSC)] and K142 [with a "red" luminescent solar concentrator (LSC)]: with the same light intensity emitted from the light source, corresponding to 865 $\mu$E/m$^2$/s, a value typical of photoinhibition, the characterization by means of Water PAM fluorometry shows tendentially higher non-photochemical quenching values (NPQ), for the test without a luminescent solar concentrator (LSC): this means that this culture has a greater tendency to protect itself from photoinhibition and disposes of the extra energy as heat, this available energy does not increase the photosynthesis yield.

With respect to the tests K145 [without a "red" luminescent solar concentrator (LSC)] and K144 [with a "red" luminescent solar concentrator (LSC)]: with the same light intensity emitted from the light source, corresponding to 616 $\mu$E/m$^2$/s, there are no significant differences in behaviour with the same light energy which reaches the photobioreactor (FBR) within the spectrum useful for photosynthesis (red + blue).

**Claims**

1. An integrated process for the cultivation of plants and the production of electric energy comprising:

   - cultivating said plants in a cultivation area comprising at least one luminescent solar concentrator (LSC) in which at least one photovoltaic cell is positioned on at least one of its outer sides, obtaining plants and electric energy;
   - recovering said plants;
   - recovering said electric energy;

   wherein said cultivation area is a greenhouse; and

   the electric energy recovered through said luminescent solar concentrator (LSC) is used in the above-mentioned process for the cultivation of plants, for example, in the management of greenhouses, in particular for the ventilation or heating of the same wherein said luminescent solar concentrator comprises at least one photoluminescent compound having an absorption range within the range of solar radiation, capable of activating photosynthesis (Photosynthetically Active Radiations - PAR.s: 400 nm - 700 nm) and an emission range capable of activating the photovoltaic cell, wherein said emission range is superimposable with respect to the maximum quantum efficiency area of the photovoltaic cell; wherein said photoluminescent compound is selected from: acene compounds; benzothiadiazole compounds; benzoheterodiazole compounds disubstituted with benzodithiophene groups; naphthoheterodiazole compounds disubstituted with benzodithiophene groups; naphthothiadiazole compounds disubstituted with thiophene groups; perylene compounds known under the trade-name of Lumogen® of Basf; wherein said luminescent solar concentrator (LSC) comprises a matrix made of transparent material selected from: transparent polymers such as polymethylmethacrylate (PMMA), polycarbonate (PC) polyisobutyl methacrylate, polyethyl methacrylate, polyallyl diglycol carbonate, polymethacrylimide, polycarbonate ether, styrene acrylonitrile, polystyrene, methyl-methacrylate styrene copolymers, polyether sulfone, polysulfone, cellulose triacetate, or mixtures thereof; transparent glass such as silica, quartz, alumina, titania, or mixtures thereof; wherein said photoluminescent compound is present in said luminescent solar concentrator (LSC) in an amount ranging from 0.1 g per surface unit to 5 g per surface unit, said surface unit referring to the surface of the matrix of transparent material expressed as m$^2$.

2. An integrated process for the cultivation of algae and the production of electric energy comprising:

- cultivating at least one alga in the presence of an aqueous culture medium in a cultivation area comprising at least one luminescent solar concentrator (LSC) in which at least one photovoltaic cell is positioned on at least one of its outer sides, obtaining an aqueous suspension of algal biomass and electric energy;
- recovering said algal biomass from said aqueous suspension of algal biomass;
- recovering said electric energy;

wherein said cultivation area is selected from: open ponds (OP), photoreactors (FR), photobioreactors (FBR) or combinations thereof; and

the electric energy recovered by means of said luminescent solar concentrator (LSC) is used in the above-mentioned process for the cultivation of algae, for example for the management of open ponds (OP), photoreactors (FR), photobioreactors (FBR), in particular for stirring the suspension of the algal biomass formed during growth, for the distribution of liquids and gas, for the functioning of the equipment for the collection, concentration and conversion of the microalgae into precursors of biofuel, either chemically or thermo-chemically wherein said luminescent solar concentrator comprises at least one photoluminescent compound having an absorption range within the range of solar radiation, capable of activating photosynthesis (Photosynthetically Active Radiations - PAR.s: 400 nm - 700 nm) and an emission range capable of activating the photovoltaic cell, wherein said emission range is superimposable with respect to the maximum quantum efficiency area of the photovoltaic cell; wherein said photoluminescent compound is selected from: acene compounds; benzothiadiazole compounds; benzoheterodiazole compounds disubstituted with benzodithiophene groups; naphthoheterodiazole compounds disubstituted with benzodithiophene groups; naphthothiadiazole compounds disubstituted with thiophene groups; perylene compounds known under the trade-name of Lumogen® of Basf; wherein said luminescent solar concentrator (LSC) comprises a matrix made of transparent material selected from: transparent polymers such as polymethylmethacrylate (PMMA), polycarbonate (PC) polyisobutyl methacrylate, polyethyl methacrylate, polyallyl diglycol carbonate, polymethacrylimide, polycarbonate ether, styrene acrylonitrile, polystyrene, methyl-methacrylate styrene copolymers, polyether sulfone, polysulfone, cellulose triacetate, or mixtures thereof; transparent glass such as silica, quartz, alumina, titania, or mixtures thereof; wherein said photoluminescent compound is present in said luminescent solar concentrator (LSC) in an amount ranging from 0.1 g per surface unit to 5 g per surface unit, said surface unit referring to the surface of the matrix of transparent material expressed as m$^2$.

3. The integrated process according to claim 1 or 2, wherein said luminescent solar concentrator (LSC) is interposed between said cultivation area and solar light.

4. The integrated process according to claim 3, wherein said luminescent solar concentrator (LSC) is interposed between said cultivation area and solar light so as to totally or partially cover said cultivation area.

5. The integrated process according to claim 1 or 2, wherein said luminescent solar concentrator (LSC) is an integral part of said cultivation area.

6. The integrated process according to claim 1, wherein said luminescent solar concentrator (LSC) forms at least partially or totally the roof or at least partially or totally the walls of said greenhouse.


**Patentansprüche**

1. Integriertes Verfahren zur Kultivierung von Pflanzen und Erzeugung von elektrischer Energie, das Folgendes umfasst:

   - Kultivieren der Pflanzen in einem Kultivierungsbereich, der mindestens einen Lumineszenz-Solarkonzentrator (LSC) umfasst, in dem mindestens eine Photovoltaikzelle an mindestens einer seiner Außenseiten angeordnet ist, Erhalten von Pflanzen und elektrischer Energie;
   - Rückgewinnen der Pflanzen;
   - Rückgewinnen der elektrischen Energie;

   wobei der Kultivierungsbereich ein Gewächshaus ist; und

   die durch den Lumineszenz-Solarkonzentrator (LSC) zurückgewonnene elektrische Energie in dem oben genannten Verfahren zur Kultivierung von Pflanzen verwendet wird, beispielsweise für die Bewirtschaftung von Gewächshäusern, insbesondere für die Belüftung oder Heizung der selbigen
   wobei der Lumineszenz-Solarkonzentrator mindestens eine photolumineszierende Verbindung umfasst, die einen Absorptionsbereich innerhalb des Bereichs der Sonnenstrahlung aufweist, der in der Lage ist, die Photosynthese zu aktivieren (Photosynthetisch Aktive Strahlung - PAR.s: 400 nm - 700 nm), und einen Emissionsbereich, der in der Lage ist, die Photovoltaikzelle zu aktivieren, wobei der Emissionsbereich sich mit der Fläche des maximalen Quantenwirkungsgrads der Photovoltaikzelle überlagern lässt;
   wobei die photolumineszierende Verbindung ausgewählt ist aus: Acenverbindungen; Benzothiadiazolverbindungen; Benzoheterodiazol-Verbindungen, die mit Benzodithiophen-Gruppen disubstituiert sind; Naphthoheterodiazol-Verbindungen, die mit Benzodithiophen-Gruppen disubstituiert sind; Naphthothiadiazol-Verbindungen, die mit Thiophen-Gruppen disubstituiert sind; Perylen-Verbindungen, die unter dem Handelsnamen Lumogen® von Basf bekannt sind;
wobei der Lumineszenz-Solarkonzentrator (LSC) eine Matrix aus einem transparenten Material umfasst, das ausgewählt ist aus: transparenten Polymeren wie Polymethylmethacrylat (PMMA), Polycarbonat (PC) Polyisobutylmethacrylat, Polyethylmethacrylat, Polyallyldiglykolcarbonat, Polymethacrylimid, Polycarbonatether, Styrol-Acrylnitril, Polystyrol, Methylmethacrylat-Styrol-Copolymeren, Polyethersulfon, Polysulfon, Cellulosetriacetat oder Mischungen davon; transparentes Glas wie Silica, Quarz, Aluminia, Titanoxid oder Mischungen davon; wobei die photolumineszierende Verbindung in dem Lumineszenz-Solarkonzentrator (LSC) in einer Menge im Bereich von 0,1 g pro Oberflächeneinheit bis 5 g pro Oberflächeneinheit vorhanden ist, wobei sich die Oberflächeneinheit auf die Oberfläche der Matrix aus transparentem Material, ausgedrückt als $m^2$, bezieht.

2. Integriertes Verfahren zur Kultivierung von Algen und Erzeugung von elektrischer Energie, das Folgendes umfasst:

   - Kultivieren mindestens einer Alge in der Gegenwart von einem wässrigen Kulturmedium in einem Kultivierungsbereich, der mindestens einen Lumineszenz-Solarkonzentrator (LSC) umfasst, in dem mindestens eine Photovoltaikzelle an mindestens einer seiner Außenseiten angeordnet ist, wobei eine wässrige Suspension von Algenbiomasse und elektrische Energie erhalten wird;
   - Rückgewinnung der Algenbiomasse aus der wässrigen Suspension von Algenbiomasse;
   - Rückgewinnung der elektrischen Energie;

   wobei der Kultivierungsbereich ausgewählt ist aus: offenen Teichen (OP), Photoreaktoren (FR), Photobioreaktoren (FBR) oder Kombinationen davon; und

   die durch den Lumineszenz-Solarkonzentrator (LSC) gewonnene elektrische Energie in dem oben genannten Verfahren zur Kultivierung von Algen verwendet wird, zum Beispiel für die Bewirtschaftung von offenen Teichen (OP), Photoreaktoren (FR), Photobioreaktoren (FBR), insbesondere für das Rühren der Suspension von der während des Wachstums gebildeten Algenbiomasse, für die Verteilung von Flüssigkeiten und Gasen, für das Funktionieren der Ausrüstung zum Sammeln, Konzentrieren und Umwandeln der Mikroalgen in Vorstufen von Biokraftstoff, entweder chemisch oder thermochemisch wobei der Lumineszenz-Solarkonzentrator mindestens eine photolumineszierende Verbindung umfasst, die einen Absorptionsbereich innerhalb des Bereichs der Sonnenstrahlung aufweist, der in der Lage ist, die Photosynthese zu aktivieren (Photosynthetisch Aktive Strahlung - PAR.s: 400 nm - 700 nm), und einen Emissionsbereich, der in der Lage ist, die Photovoltaikzelle zu aktivieren, wobei der Emissionsbereich sich mit der Fläche des maximalen Quantenwirkungsgrads der Photovoltaikzelle überlagern lässt;

wobei die photolumineszierende Verbindung ausgewählt ist aus: Acenverbindungen; Benzothiadiazolverbindungen; Benzoheterodiazol-Verbindungen, die mit Benzodithiophen-Gruppen disubstituiert sind; Naphthoheterodiazol-Verbindungen, die mit Benzodithiophen-Gruppen disubstituiert sind; Naphthothiadiazol-Verbindungen, die mit Thiophen-Gruppen disubstituiert sind; Perylen-Verbindungen, die unter dem Handelsnamen Lumogen® von Basf bekannt sind;

wobei der Lumineszenz-Solarkonzentrator (LSC) eine Matrix umfasst die aus einem transparenten Material gemacht ist, das ausgewählt ist aus: transparenten Polymeren wie Polymethylmethacrylat (PMMA), Polycarbonat (PC), Polyisobutylmethacrylat, Polyethylmethacrylat, Polyallyldiglykolcarbonat, Polymethacrylimid, Polycarbonatether, Styrolacrylnitril, Polystyrol, Methylmethacrylat-Styrol-Copolymeren, Polyethersulfon, Polysulfon, Cellulosetriacetat, oder Mischungen davon; transparentes Glas wie Silica, Quarz, Alumina, Titanoxid oder Mischungen davon;

wobei die photolumineszierende Verbindung in dem Lumineszenz-Solarkonzentrator (LSC) in einer Menge von 0,1 g pro Oberflächeneinheit bis 5 g pro Oberflächeneinheit vorhanden ist, wobei sich die Oberflächeneinheit auf die Oberfläche der Matrix aus transparentem Material, ausgedrückt als $m^2$, bezieht.

**3.** Integriertes Verfahren gemäß Anspruch 1 oder 2, wobei der Lumineszenz-Solarkonzentrator (LSC) zwischen dem Kultivierungsbereich und Sonnenlicht angeordnet ist.

**4.** Integriertes Verfahren gemäß Anspruch 3, wobei der Lumineszenz-Solarkonzentrator (LSC) zwischen dem Kultivierungsbereich und dem Sonnenlicht so angeordnet ist, dass er den Kultivierungsbereich ganz oder teilweise bedeckt.

**5.** Integriertes Verfahren gemäß Anspruch 1 oder 2, wobei der Lumineszenz-Solarkonzentrator (LSC) ein integraler Bestandteil des Kulturbereichs ist.

**6.** Integriertes Verfahren gemäß Anspruch 1, wobei der Lumineszenz-Solarkonzentrator (LSC) zumindest teilweise oder vollständig das Dach oder zumindest teilweise oder vollständig die Wände des Gewächshauses bildet.

## Revendications

**1.** Procédé intégré pour la culture de plantes et la production d'énergie électrique comprenant les étapes suivantes:

- cultiver lesdites plantes dans une zone de culture comprenant au moins un concentrateur solaire luminescent (LSC) dans lequel au moins une cellule photovoltaïque est positionnée sur au moins l'un de ses côtés extérieurs, obtenant des plantes et de l'énergie électrique;
- récupérer lesdites plantes;
- récupérer ladite énergie électrique;

danslequel ladite zone de culture est une serre; et

l'énergie électrique récupérée par l'intermédiaire dudit concentrateur solaire luminescent (LSC) est utilisée dans le procédé susmentionné pour la culture de plantes, par exemple dans la gestion de serres, notamment pour la ventilation ou le chauffage de ces dernières;

dans lequel ledit concentrateur solaire luminescent comprend au moins un composé photoluminescent ayant une bande d'absorption comprise dans la bande du rayonnement solaire, capable d'activer la photosynthèse (rayonnement de photosynthèse, ou PAR.s: 400 nm - 700 nm) et une bande d'émission capable d'activer la cellule photovoltaïque, dans lequel ladite bande d'émission est superposable par rapport à la zone de rendement quantique maximum de la cellule photovoltaïque;

dans lequel ledit composé photoluminescent est choisi parmi: les composés d'acène ; les composés de benzothiadiazole; les composés de benzohétérodiazole disubstitués avec des groupes benzodithiophène; les composés de naphtohétérodiazole disubstitués avec des groupes benzodithiophène ; les composés de naphtothiadiazole disubstitués avec des groupes thiophène ; les composés de pérylène connus sous l'appellation commerciale Lumogen® de Basf;

dans lequel ledit concentrateur solaire luminescent (LSC) comprend une matrice faite d'un matériau transparent choisi parmi: les polymères transparents comme le polyméthacrylate de méthyle (PMMA), le polycarbonate (PC), le polyméthacrylate d'isobutyle, le polyméthacrylate d'éthyle, le polyallyl diglycol carbonate, le polyméthacrylimide, le polycarbonate éther, le styrène-acrylonitrile, le polystyrène, les copolymères méthacrylate de méthyle-styrène, le polyéthersulfone, la polysulfone, le triacétate de cellulose, ou leurs mélanges ; un verre transparent tel que la silice, le quartz, l'alumine, le dioxyde de titane, ou leurs mélanges;

dans lequel ledit composé photoluminescent est présent dans ledit concentrateur solaire luminescent (LSC) en une quantité allant de 0,1 g par unité de surface à 5 g par unité de surface, ladite unité de surface se rapportant à la surface de la matrice de matériau transparent exprimée en m$^2$.

2. Procédé intégré pour la culture d'algues et la production d'énergie électrique comprenant les étapes suivantes:

- cultiver au moins une algue en présence d'un milieu de culture aqueux dans une zone de culture comprenant au moins un concentrateur solaire luminescent (LSC) dans lequel au moins une cellule photovoltaïque est positionnée sur au moins l'un de ses côtés extérieurs, obtenant une suspension aqueuse de biomasse algale et de l'énergie électrique;
- récupérer ladite biomasse algale de ladite suspension aqueuse de biomasse algale;
- récupérer ladite énergie électrique;

dans lequel ladite zone de culture est choisie parmi : les bassins ouverts (OP), les photoréacteurs (FR), les photobioréacteurs (FBR) ou leurs combinaisons; et

l'énergie électrique récupérée au moyen dudit concentrateur solaire luminescent (LSC) est utilisée dans le procédé susmentionné pour la culture d'algues, par exemple pour la gestion de bassins ouverts (OP), de photoréacteurs (FR), de photobioréacteurs (FBR), notamment pour brasser la suspension de la biomasse algale formée pendant la croissance, pour la distribution de liquides et de gaz, pour le fonctionnement de l'installation pour la collecte, la concentration et la conversion des micro-algues en précurseurs de biocarburant, par voie chimique ou thermochimique;

dans lequel ledit concentrateur solaire luminescent comprend au moins un composé photoluminescent ayant une bande d'absorption comprise dans la bande du rayonnement solaire, capable d'activer la photosynthèse (rayonnement de photosynthèse, ou PAR.s: 400 nm - 700 nm) et une bande d'émission capable d'activer la cellule photovoltaïque, dans lequel ladite bande d'émission est superposable par rapport à la zone de rendement quantique maximum de la cellule photovoltaïque;

dans lequel ledit composé photoluminescent est choisi parmi : les composés d'acène ; les composés de benzothiadiazole; les composés de benzohétérodiazole disubstitués avec des groupes benzodithiophène; les composés de naphtohétérodiazole disubstitués avec des groupes benzodithiophène ; les composés de naphtothiadiazole disubstitués avec des groupes thiophène ; les composés de pérylène connus sous l'appellation commerciale Lumogen® de Basf;

dans lequel ledit concentrateur solaire luminescent (LSC) comprend une matrice faite d'un matériau transparent choisi parmi : les polymères transparents comme le polyméthacrylate de méthyle (PMMA), le polycarbonate (PC), le polyméthacrylate d'isobutyle, le polyméthacrylate d'éthyle, le polyallyl diglycol carbonate, le polyméthacrylimide, le polycarbonate éther, le styrène-acrylonitrile, le polystyrène, les copolymères méthacrylate de méthyle-styrène, le polyéthersulfone, la polysulfone, le triacétate de cellulose, ou leurs mélanges ; un verre transparent tel que la silice, le quartz, l'alumine, le dioxyde de titane, ou leurs mélanges;

dans lequel ledit composé photoluminescent est présent dans ledit concentrateur solaire luminescent (LSC) en une quantité allant de 0,1 g par unité de surface à 5 g par unité de surface, ladite unité de surface se rapportant à la surface de la matrice de matériau transparent exprimée en m$^2$.

3. Procédé intégré selon la revendication 1 ou 2, dans lequel ledit concentrateur solaire luminescent (LSC) est intercalé entre ladite zone de culture et la lumière solaire.

4. Procédé intégré selon la revendication 3, dans lequel ledit concentrateur solaire luminescent (LSC) est intercalé entre ladite zone de culture et la lumière solaire afin de couvrir totalement ou partiellement ladite zone de culture.

5. Procédé intégré selon la revendication 1 ou 2, dans lequel ledit concentrateur solaire luminescent (LSC) fait partie intégrante de ladite zone de culture.

6. Procédé intégré selon la revendication 1, dans lequel ledit concentrateur solaire luminescent (LSC) forme au moins partiellement ou totalement le toit ou au moins partiellement ou totalement les parois de ladite serre.

# Fig. 1

Photosynthesis yield ("Yield") at the beginning of the exposure period

# Fig. 2

Photosynthesis yield ("Yield") at the end of the exposure period (20 days)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110281295 A
- US 20120034679 A
- WO 2011048458 A
- WO 2012007834 A
- IT MI20130605
- IT MI20130606
- EP 1728844 A

**Non-patent literature cited in the description**

- **ANTAL T. et al.** *International Journal of Hydrogen Energy,* 2003, vol. 37, 8859-8863
- **HAMMAN M.** *Desalination,* 2007, vol. 209, 244-250